# EUROPEAN PATENT SPECIFICATION

(11) **EP 2 985 040 B1**
(45) Date of publication and mention of the grant of the patent: **06.03.2019**
(21) Application number: 15168592.2
(22) Date of filing: 09.10.2009
(51) Int. Cl.: A61L 2/26, B65B 61/14, B65D 75/56, B65D 71/00

(54) **STERILISATION ASSEMBLY FOR ORTHOPAEDIC DEVICE**
STERILISATIONSANORDNUNG FÜR ORTHOPÄDISCHE GERÄTE
ENSEMBLE DE STÉRILISATION POUR DISPOSITIF ORTHOPÉDIQUE

(30) Priority: 10.10.2008 US 104302 P
(43) Date of publication of application: 17.02.2016
(62) Divisional of application: 09819972.2
(73) Proprietor: DePuy Ireland Unlimited Company, County Cork (IE)
(72) Inventor: Thomas, Kyle, Denver, CO Colorado 80212 (US); Reeve, Michael, Leeds, Yorkshire LS11 8DT (GB); Kecman, Maja, London, Greater London E2 7LJ (GB); Stroux, Lisa, 69120 Heidelberg (DE); Pell, Randy, Marlboro, VT Vermont 05344 (US); Bentley, Dan, Rochester, NY New York 14623 (US)
(74) Representative: Alton, Andrew

(56) References cited:
- EP-A2- 0 745 536
- WO-A1-2006/055083
- WO-A2-2009/018948
- DE-A1- 4 238 535
- US-B1- 7 021 485

## Description

The present invention relates to a cover, and in particular to a cover for sterilisable medical containers to improve the handling, storage and organisation of such containers after sterilisation.

Reusable medical devices, such as instruments, need to be sterilised before re-use. Often steam based sterilisation systems are used to sterilise the instruments. Sterilisation systems typically include a container, such as a rigid exterior perforated case of either a metal or plastic material and having a rigid lid. Instruments are often held in a tray which is placed in the case. Multiple trays can be stacked within the same case. Common sterilisation practices require the cases to be wrapped in a sheet or sheets of a wrapping material which provide a sterile barrier to maintain the sterility of the instruments after sterilisation. The wrapping material, sometime referred to as "bio-wrap", is often provided as sheets of paper of fabric and one or more sheets of the wrap are used to wrap the cases prior to sterilisation so that the outer wrapping maintains the sterility of the cases post sterilisation.. This can create a large amount of disposable waste.

The sterilised wrapped cases are then placed in hospital storage rooms until transport to the operating room is required. However, the wraps are susceptible to rips and tears which compromise the sterility of the instruments within the case. Also, the uneven wraps tend to make it difficult to stack and organise the cases on the shelves in the storage rooms. Sometimes, when trying to remove a case from a stack of cases, the wrap around the case being removed or other cases in the stack can be damaged thereby compromising sterility. Also, other cases in the stack or in adjoining stacks can be dislodged from the shelves and fall to the floor again damaging the wrap and sometimes spilling the content of the cases. Handles placed on the exterior of cases are not accessible when the tray is wrapped. The wrapping material makes it difficult to transport wrapped containers and the lack of proper handles leads to ripping of the wrap. In an attempt to clasp onto the handle mechanism of the case under the wrap, fingers can penetrate the wrap and again compromise the sterility of the instrument case. Whenever the sterility has been compromised or possibly compromised, it is necessary to re-sterilise the case and contents which is a drain on the sterilisation resources available to the hospital and can lead to understocking of sterilised instruments available for use.

Attempts at ameliorating these problems have focussed on removing the necessity for the use of bio-wraps through the use of a sterile container or a disposable filter bag in place of the wrapping material. However, many sterilisation facilities still use the practice of wraps to maintain sterility during storage and transportation.

WO 2006/055083 describes a sterilization wrap for a sterilisable tray holding instruments. An adhesive on the surface of the sterilization wap activates upon exposure to sterilization conditions to hold the sterilizatio wrap in a closed configuration about the tray.

US 7,021, 485 describes a modular sterilisable tray for medical devices, WO 2009/018948 describes a sterilization method for groups of objects, DE 4238535 describes a clamping device for retaining packed glass containers on a tray for sterilisation and EP 0745536 describes an apparatus and method for the sterilization of blister packages of contact lenses and secondary packaging into cartons.

Therefore there is a need to improve the maintenance of sterility during storage and handling of wrapped sterile medical containers.

According to a first aspect of the invention, there is provided an assembly comprising: a sterilizable medical tray; a medical device in the medical tray; wrapping material wrapped around the medical tray; and characterised in that the assembly further comprises: a cover around the wrapped medical tray to hold the wrapping material about the medical tray, wherein the cover includes: a body including an upper part holding the wrapping material against an upper portion of the medical tray, a lower part holding the wrapping material against a lower portion of the medical tray; and at least one handle extending from the body, and provided as an integral part of the body, and which can be held by a user to move the assembly, and wherein the cover is made from a single sheet of sterilizable material and is assembled about the wrapped sterilizable tray by folding the sheet of material and the medical device is an orthopaedic implant, orthopaedic instrument or orthopaedic tool.

A cover goes around the outside of the wrapped container which acts to hold the wrap against the container and helps protect the wrap from damage and also includes a handle to facilitate handling of the wrapped container. Hence, the cover helps to reduce damage to the wrap material during storage and handling of the sterile containers and thereby helping to maintain their sterility and reduce the number of containers that need to be re-sterilised.

By at least partially covering the wrapping material, damage to the wrapping material can be reduced thereby helping to maintain the sterility of the container and its contents after sterilisation.

By holding the wrapping material against the container, the cover also to keep the wrapped container more tidy, which helps to improve the handling, storage and manipulation of the covered container, thereby also helping to reduce damage to the wrapping material and hence improving sterility.

Further, by providing a handle as part of the cover, a user can use the handle to manipulate and mover the covered wrapped container and so the user does not need to handle the wrapping material itself, which yet further helps to reduce damage to the wrapping material and hence helps to maintain sterility.

The body can have a generally rectangular shape and the handle can be provided on the shorter side of the body or on the longer side of the body.

The cover can include a plurality of handles. Preferably the cover can includes two or at least two handles. A first handle can be provided at a first end of the body. A second handle can be provided at a second end of the body. The second end can be at an end opposite to the first end. A handle can be provided at each end of the body and at the ends separated by the longest dimension of the body.

The body can be permeable to steam. The material of the body can be made of a material which is itself permeable to steam. The body can include a plurality of apertures to make the body permeable to steam. This facilitates the ingress and egress of steam during sterilisation when the cover is made of a material which is not itself permeable to steam.

The cover can include a fastener for securing the cover about the wrapped container. The fastener can secure the upper member and lower member together when the cover is assembled about the wrapped container. The fastener can be a single use fastener or the fastener can be a releasable fastener. The fastener can be a mechanical fastener or can be an adhesive fastener.

The sheet of material can be engineered to cause the sheet to fold along preferred lines or directions. The sheet of material can include at least one or a plurality of fold formation which define where the sheet of material can be folded to assemble the cover. The fold formations can be positioned on the sheet to define the upper and lower portions and at least a first side portion and preferably also a second side portion. The fold formations can be positioned so that the cover when assembled will snugly accept a wrapped sterilisable medical container.

The cover can be made from a cardboard. The cover can includes an adhesive material positioned on the cover so that the adhesive material can be used to secure the cover about the wrapped container when the cover is assembled about the wrapped container.

The cover can be made from a plastic material.

The sheet of material can include cuts which help to define how the sheet of material can be folded to assemble the cover.

The cover can include at least one retaining formation to help retain the wrapped container within the cover. The or each retaining formation can be located toward an open end of the cover. The or each retaining formation can act to prevent the wrapped container from escaping from the cover via the open end of the cover. The retaining formation can be provided as an integral part of the cover. The retaining formation can be formed by bending or folding a part of the material of the cover. The or each retaining formation can be defined by at least one cut line or fold formation. The or each retaining formation can be located adjacent the handle at an end of the cover. Preferably at least one retaining formation is provided at each open end of the cover.

Instrument cases are often marked with information concerning the interior contents. After the case is wrapped this information is hidden. Hospital staff sometimes create labels to mark the interior contents of the wrapped container, however this manual process is tedious and prone to error. Therefore, the cover can bear one or more indicia indicating information relating to the contents of the container. The information can relate to a manufacturer of the contents, a model name of the contents, the type of the contents, the size of the contents, other attributes of the contents and any combination thereof.

The cover can be generally dimensioned to receive a sterilisable medical container when at least partially covered by a wrapping material. The cover can be dimensioned to receive a container having dimensions in metres of height, width and depth selected from:0.05 by 0.25 by 0.50; 0.10 by 0.25 by 0.50; 0.15 by 0.25 by 0.50; 0.05 by 0.25 by 0.25; and 0.10 by 0.25 by 0.25.

A further aspect of the invention provides a method for covering a sterilisable medical container wrapped in at least one sheet of wrapping material, comprising the steps of: placing a sterilizable medical tray, having an orthopaedic implant, instrument or tool therein, and wrapped in the wrapping material onto a lower part of a body of the cover to hold the wrapping material against a lower portion of the medical tray; and folding the body of the cover about the wrapped medical container so that an upper part of the body of the cover holds the wrapping material against an upper portion of the medical tray so as to hold the wrapping material about the medical tray; and securing the cover about the wrapped medical tray, wherein the cover is made from a single sheet of sterilizable material and includes at least one handle extending from the body, and provided as an integral part of the body, and which can be held by a user to move an assembly of the covered sterilizable medical tray wrapped in at least one sheet of wrapping material.

Embodiments of the invention will now be described, by way of example only, and with reference to the accompanying drawings, in which:
Figure 1 shows a plan view of a first embodiment of a cover according to the invention in the form of a cardboard foldable flat;
Figures 2A to 2D illustrate assembly of the cover shown in Figure 1 about a wrapped container;
Figure 3 shows a perspective view of a second embodiment of a cover according to the invention;
Figure 4 shows a perspective view of a third embodiment of a cover according to the invention;
Figure 5 shows a plan view of a fourth embodiment of a cover according to the invention in the form of a plastics foldable flat;
Figure 6 shows a plan view of a fifth embodiment of a cover according to the invention in the form of a plastics foldable flat;
Figure 7 shows a magnified view of a corner feature of the cover shown in Figure 6, when assembled in use;
Figure 8 shows a plan view of a sixth embodiment of a cover according to the invention in the form of a plastics foldable flat;
Figure 9 shows a magnified view of a corner feature of the cover shown in Figure 8, when assembled in use;
Figure 10 shows a perspective view of an example_cover when assembled in use;
Figure 11 shows a magnified view of a fastener feature of the cover shown in Figure 10; and
Figure 12 shows a plan view of a component part of the cover shown in Figures 10 and 11.

Similar items in different Figures share common reference numerals unless indicated otherwise.

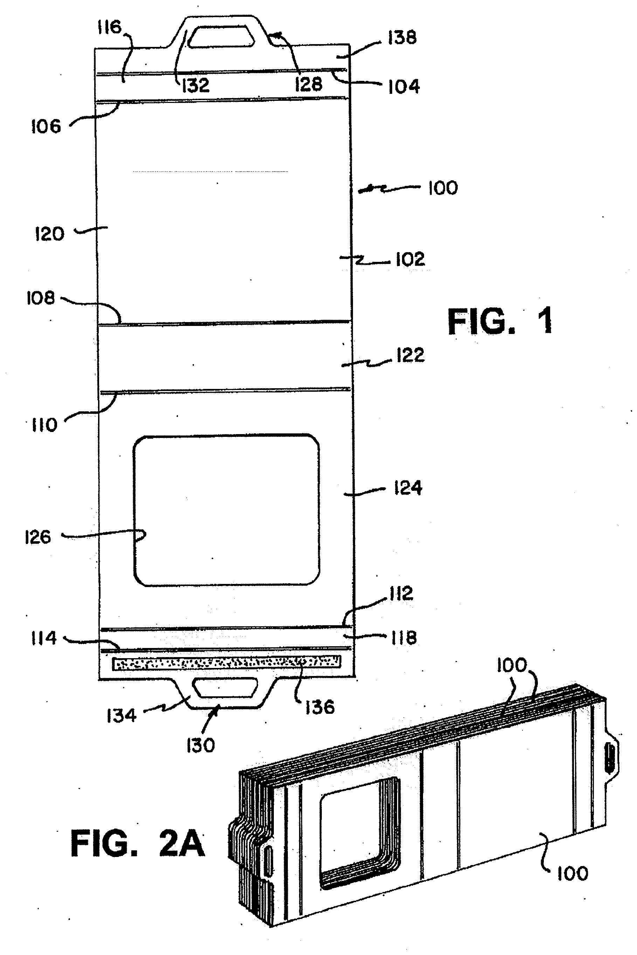

With reference to Figure 1 there is shown a first embodiment of a cover 100 for assembling about a wrapped sterilisable container. The container may be a case or similar, or simply a tray holding whatever is to be sterilised. In the following, the container will generally be used to refer to any structure which is used to hold or support whatever is being sterilised during the sterilisation process. For example, the container may refer to a case with a one or more trays located therein or simply to a sterilisable tray, or similar, or multiple trays or similar.

The cover 100 is in the form of a foldable flat 102 made of a cardboard material. In other embodiments, the foldable flap may be made from a plastic material.

The foldable flat 102 includes a number of fold formations 104, being parts of the cardboard flat which have been engineered are otherwise adapted to cause the cardboard material to preferentially fold along that line, in a desired direction. The engineered folds 104 to 114 are positioned on the flat so as to define portions corresponding to first and second side walls, and upper and lower portions or surfaces of the container to be covered. For example, portions 116 and 118 are dimensioned to provide a cover for a first side wall, portion 122 a cover for a second side wall, portion 120 a cover for a lower portion of the container and portion 124 a cover for an upper portion of the container. Portion 124 also includes an aperture 126 therein which provides a window via which information about the contents of the container on a printed sheet can be viewed.

A first handle portion 128 and a second handle portion 130 are provided at first and second ends of the flat respectively. Each handle portion includes a handle formation 132, 134 which co-operate and assembled to provide a handle for the cover. Handle portion 130 also includes a strip of adhesive material 136. The adhesive material can be used to provide a fastener to secure the cardboard flat in its assembled configuration when assembled about a wrap container in use. Adhesive material 136 can be a contact adhesive which adheres to a corresponding part 138 of handle portion 128. In alternative embodiments, adhesive 136 may be covered by a releasable cover strip which is removed prior to assembly of the cover 100.

As described above, it will be appreciated that the cover generally has a main body section with an integral handle formations 132 and 134. The foldable flap 102 can easily be manufactured simply by a stamping process in which the apertures are created by a cutting instrument acting on a sheet of cardboard material and in which the fold lines 104 to 114 are created by a scoring, or similar, process. In an alternate embodiment, portions of the body of the foldable flap 102 are provided with a plurality of apertures arranged generally all over the body in order to allow the ingress and egress of steam during sterilization.

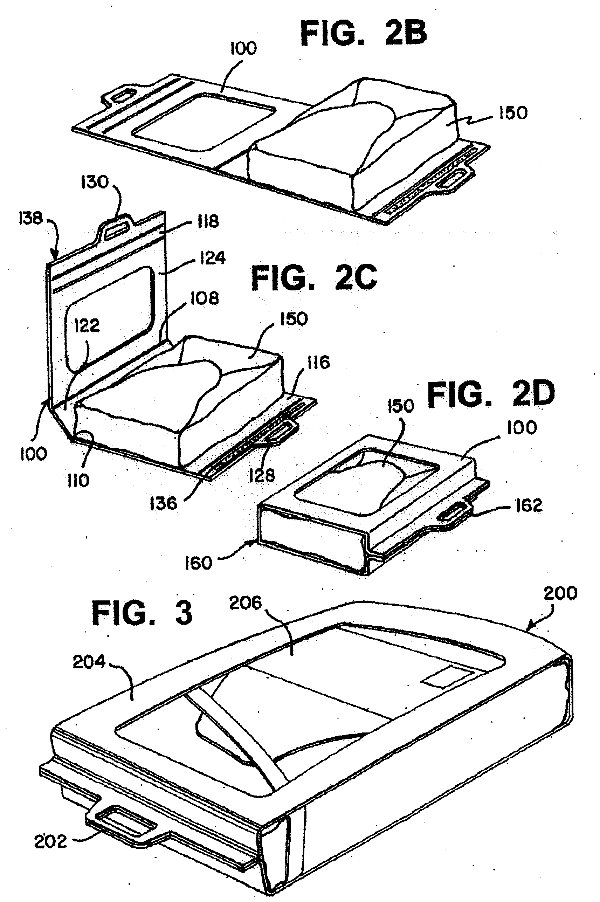

With reference to Figures 2A to 2D, use of the cover 100 will be described in greater detail. Figure 2A shows a plurality of covers 100 as illustrated in Figure 1. A cover 100 in an unassembled state is selected and the sterilized container covered by at least one sheet of wrapping material 150 is placed on the lower part 120 of the cover 100, as illustrated in Figure 2B. As illustrated in Figure 2C, assembly of the cover is commenced by folding the cover along fold line 108 and 110 so that portion 122 of the cover engages a rear side of the wrapped container and eventually, by further folding cover along line 110, the upper portion 124 of the cover is brought into contact with the upper portion of the container. Portions 116 and 118 of the cover are folded down to engage a forward side of the wrapped container and handle portions 128 and 130 are folded and engaged together and adhesive 136 is brought into engagement with region 138 and thereby secures the cover in its assembled formation about the wrapped container as illustrated in Figure 2D. Hence, the end result is an assembly 160 comprising the cover 100 assembled about the wrapped container 150 as illustrated in Figure 2D.

As can be seen, the cover holds the sheet of wrapping material about the container and also at least partially protects the wrapping material by generally enclosing it. The cover 100 also presents a handle 162 by which the container can be more easily handled during storage or movement by a user.

As illustrated in Figure 1, in the first embodiment, the cover 100 has a generally rectangular shape and the handle extends along a one of the longer sides of the cover. Figure 3 shows a second embodiment of the cover 200, generally similar to the first embodiment shown in Figure 1. Cover 200 also has a generally rectangular shape, but handle 202 extends along and from a shorter side of the cover 200. It was also illustrated in Figure 3, aperture 204 in the upper part of cover 200 provides a window by which a user can view information about the contents of the sterilized container presented on a sheet of paper 206.

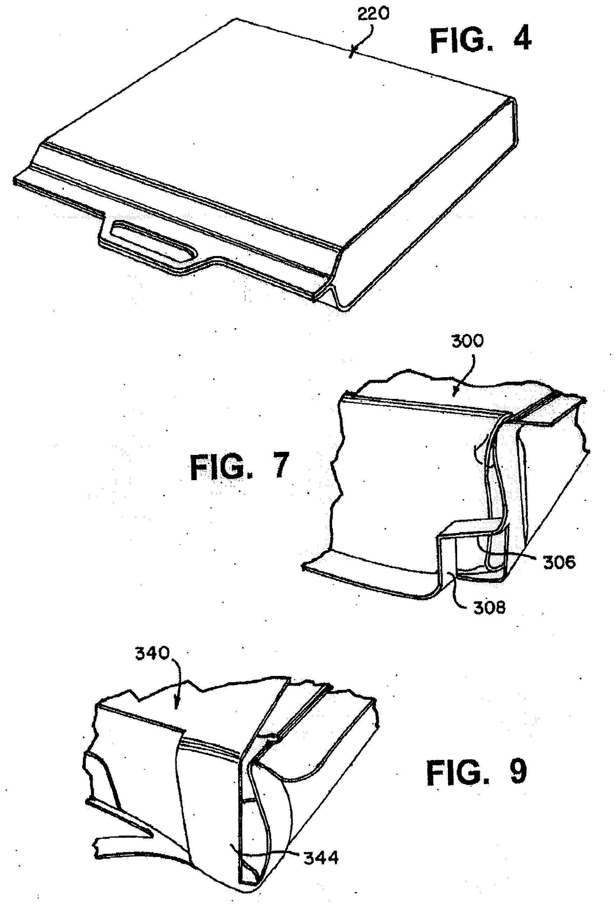

Figure 4 illustrates a third embodiment 220 of the cover. This embodiment is similar to the first embodiment except that it does not include a window aperture. As also illustrated in Figure 4, the cover can bear at least one indicium on an outer surface of the cover 220 which provides information about the content of the container. For example, the cover 220 can include an indication of the manufacturer of the contents, the type or nature of the contents, for example, the name or type of an instrument, information about the size of the contents and/or any other properties or attributes of the medial instruments or devices held in the container.

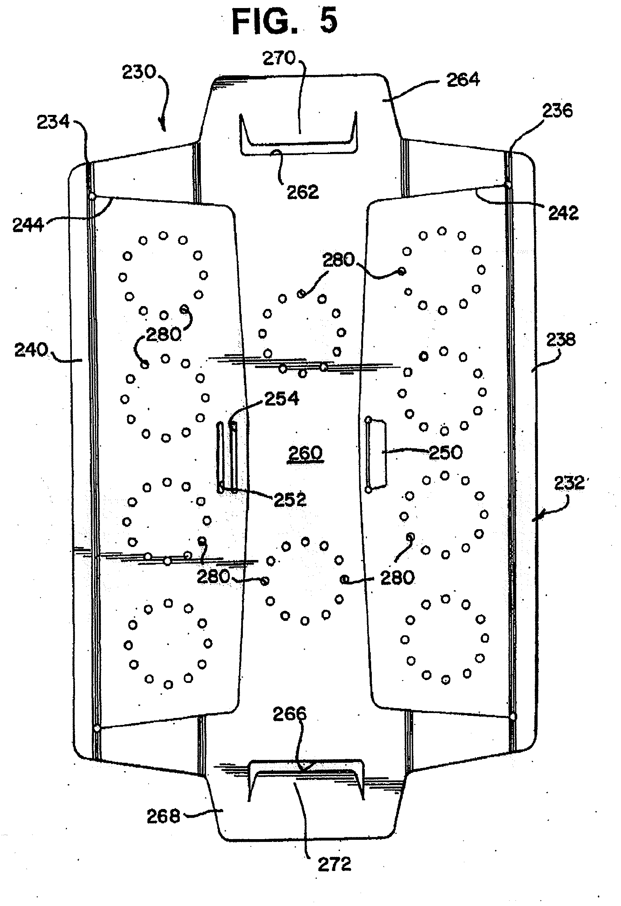

With reference to Figure 5, there is shown a plan view of a fourth embodiment 230 of a cover according to the invention. The cover 230 is in the form of a foldable flat 232 made of a plastics material, such as polypropylene, or a thermoformable material or plastic The cover 230 is shown in an unassembled state and is assembled about a wrap container in use, as will be described in greater detail below.

The plastics flat 232 includes a number of apertures and cuts in the plastics material and also includes engineered fold formations, e.g. 234, 236 to allow the flat to be folded along preferred directions. A first lip 238 extends along a first side of the flat and a second lip 240 extends along a second posed side of the flat. A first cut line 242 extends from fold formation 236 at one end of the flat to the other opposed end of the flat and defines a first half of an upper portion of the cover. A second similar cut 244 extends from fold formation 234 generally and defines a second half of the upper portion of the cover 248. The first half 246 includes a cut defining a flap or tongue member 250. Second half 248 includes a pair of slots 252, 254, positioned to receive flap 250 when the cover is assembled about a wrap container in use. Between them, flap 250 and slots 252, 254 provide a fastener for folding the cover about a wrapped container.

A central portion 260 of the flat 232 provides a lower portion of the cover for engaging a lower part of the wrapped container in use.

A first generally U-shaped aperture 262 is provided in a handle portion 264 at a first end of the flat. A second generally U-shaped aperture 266 is provided in a second handle portion 268 at a second end of the flat. U-shaped apertures 262, 266 define respective flaps 270, 272 which can be folded out of the plane of the flat 232 to provide hand holds for a user to grip a handle provided by the end handle portions 264, 268 of the flat.

The flat includes a plurality of apertures e.g. 280, arranged in ten circular patterns about the flat so as to allow the ingress and egress of steam through the material of the flat during sterilization.

In use, the first and second halves 246, 248 of the upper portion of the cover are folded out of the plane of a flat along fold formations 234 and 236 and the wrap container is placed on the lower part 260 of the cover between the first and second halves 246, 248. The first and second halves are then drawn toward each other which causes the flat to become assembled and wrap around the wrapped container and flap 250 is inserted into slot 252 or 254 in order to fasten the first and second halves 246, 248 of the flat to form the upper portion of the cover on the upper part of the wrapped container.

Hence, in this assembled state, the cover generally wraps around the wrapped container so as to hold the wrap against the container and also generally protect the wrap from damage.

In order to facilitate handling of the covered wrapped container, a user folds flaps 272, 270 to insert their hands into hand holds in the handle portions and can then transport the covered wrapped container to a sterilization facility for sterilization. The presence of the plurality of apertures, e.g. 280, allows the easy passage of steam into and out of the container via the wrap material. After sterilization, the handles can again be used by a user to facilitate handling of the sterilized container and contents and the sterilized container and contents within the wrap and cover can be transported to and stored in a storage facility until required in future. As will be appreciated, the cover helps to manage the fabric wrap and also provides substantially flat surfaces to facilitate stacking of multiple covered wrap containers in an easy manner. Also, the provision of handles allows a user to easily select the covered wrapped container from a stack of such containers and handle the container without causing damage to the wraps of other similar containers.

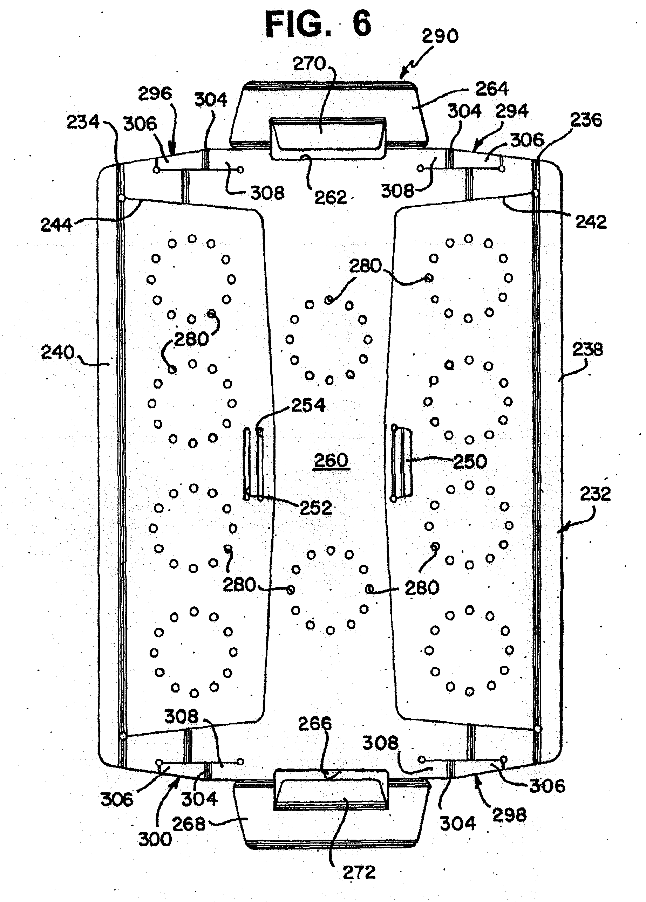

With reference to Figure 6, there is shown a fifth embodiment of a cover 290 according to the present invention. The fifth embodiment 290 is generally similar to the fourth embodiment 230 and is also provided in a form of a foldable plastics flat 292. Figure 6 shows further fold formations not necessarily shown in Figure 5. The flat also includes a mechanism to retain the wrapped within the foldable flat to prevent the container sliding out of the cover along the longitudinal axis of the cover. The mechanism is illustrated in greater detail in Figure 7 which shows a perspective view of the cover 290 when assembled in use about a wrapped container 300. A illustrated best in Figure 6, towards each of the four corners or shoulders of the flat 294, 296, 298, 300, a retaining formation is provided by a cut and a fold formation, e.g. cut 302 and fold formation 304. When the cover is assembled about the wrapped container, generally as described above, the retaining formation is provided by folding a strip of plastics material about fold formation 304 so as to provide a barrier extending from the lower part of the cover to the side wall of the cover so as to prevent any movement of the wrap container along the longitudinal axis of the cover.

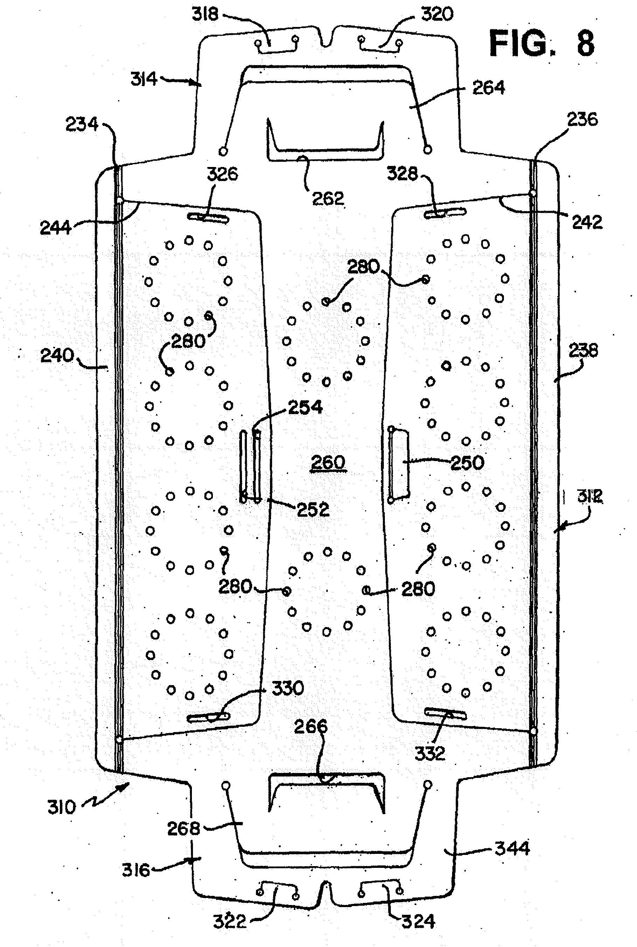

Figure 8 shows a plan view of a sixth embodiment of the cover 310 generally similar to the embodiments shown in Figures 5 and 6. Again, the cover 310 is provided in the form of a foldable plastics flat 312 and is shown in Figure 8 in an unassembled configuration. Cover 310 is similar to cover 290 in that it includes a retention mechanism to help prevent longitudinal sliding of the wrapped container within the cover in use. A retention mechanism is provided at each end of the cover. The retention mechanism takes the form of a generally U-shaped portion 314, 316 of the flat which can be folded up from the central portion 260 of the flat and which each bears a pair of tongues 318, 320, 322, 324. A slot 326, 328, 330, 322 is provided toward each end of each half of the upper portion of the cover for receiving a one of the tongues in use.

Figure 9 shows a magnified perspective view of a corner of the cover 310 when assembled in use about a wrapped container 340. After the first and second halves of the upper portion have been drawn together and fastened by tongue 250 in one of slots 252, 254, a retaining mechanism is provided by folding the U-shaped members 314, 316 upward out of the plane of the flat and inserting tongues 318 and 320 in slots 326 and 328 respectively and tongues 322, 324 in slots 330 and 332 respectively. By doing so, end retaining members, e.g. portion 344 provides a strap which engages about the end of the wrapped container to restrict its movement along the longitudinal axis of the cover.

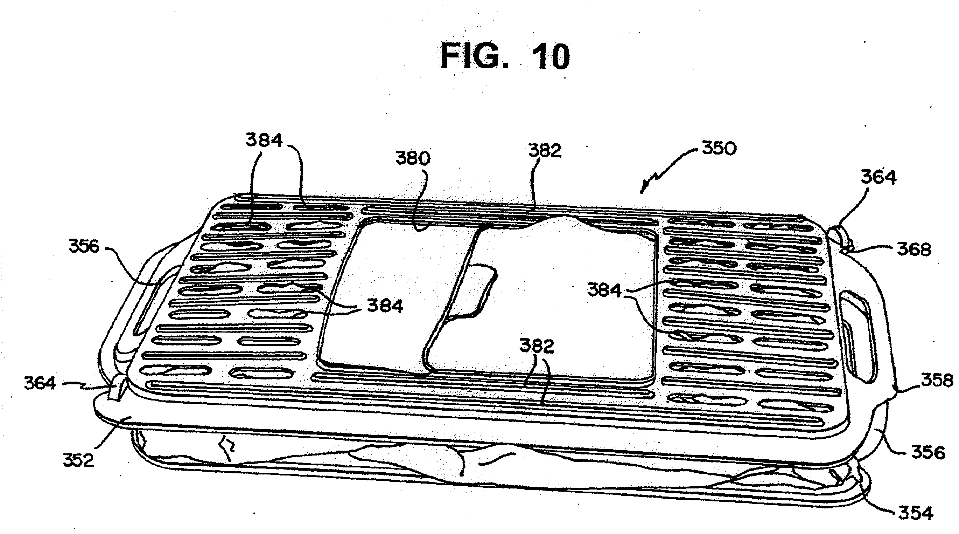

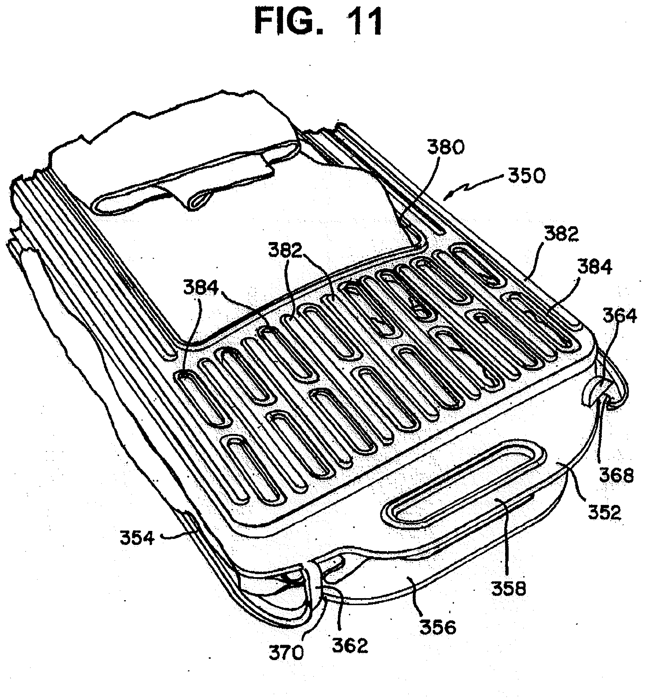

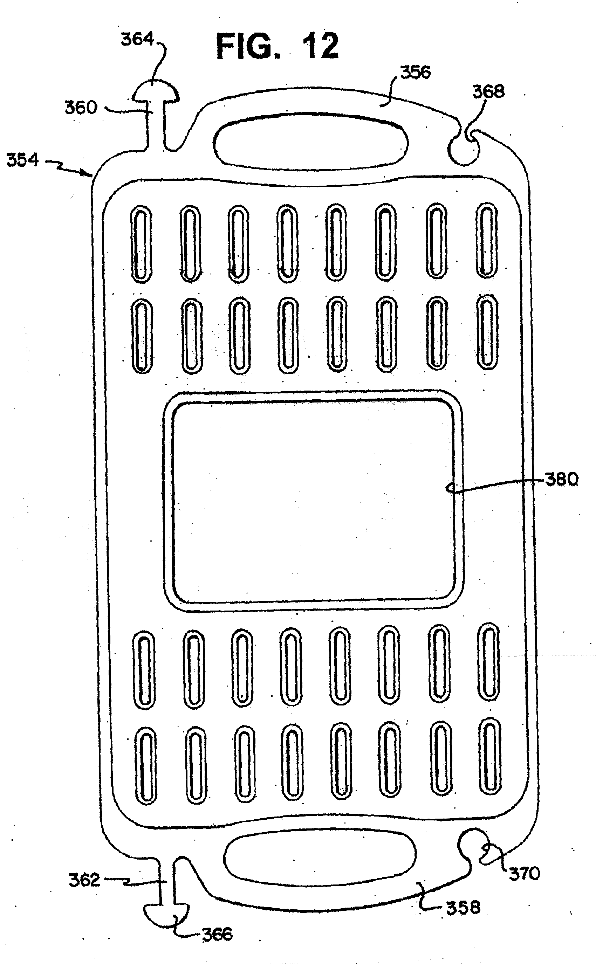

With reference to Figures 10 and 11 there are shown perspective views of an example cover 350. The cover 350 is in the form of a pair of generally tray-shaped members 352, 354, a one of which is schematically illustrated in plan view in Figure 12. Each generally tray-shaped member 352, 354 has a main generally flat section with a short wall extending up from and around the periphery thereof. A handle portion 356, 358 extends from each end of the main body of the cover. At each end, to a first side of the handle portion, a first part of a fastening mechanism is provided in the form of a strap 360, 362, terminating in a head portion 364, 366. To the opposite side of each handle portion, a generally C-shaped or re-entrant aperture 368, 370 is provided with a narrow entrance. The aperture provides a second part of the fastener mechanism and is configured to receive and retain therein the strap portion of the fastening mechanism when assembled about a wrapped container in use.

The main body of the cover includes a central large rectangular aperture 380 which provides a window via which information can be viewed. The main body of the cover also includes a plurality of lozenge or oval shaped apertures to allow the ingress and egress of steam during sterilisation. As best illustrated in Figures 10 and 11, the sequence of convex ribs and concave grooves are provided on the outer surface of each tray member. The rib and grooves alternate and are dimensioned so that a rib from a one cover will mate with a groove from another cover on which it is stacked so as to allow the stacked covered containers to be slid laterally relative to each other but to prevent sliding in a direction generally transverse to the longitudinal axis of the cover, as will be described in greater detail below.

The cover is made up from two identical tray portions, as illustrated in Figure 12, with a first of the tray portions receiving the wrapped container therein and a second tray portion inverted and placed over the top part of the wrap container as illustrated in Figures 10 and 11. Between them the tray parts provide the main body of the wrapper with the handle formations providing a handle at each end of the cover which is integral to the main body of the cover. The tray members are identical and so can be used inter-changeably in a modular fashion. Hence, it is only necessary to manufacture one type of member which can then be used as either the upper or lower part of the cover. The parts of the cover are fastened together in the assembled state by engaging stems 360, 362 in corresponding apertures in the upper inverted tray and engaging similar stems in the upper inverted tray into the apertures 368, 370 in the lower tray.

Use of cover 350 is generally similar to use of the embodiments as described above. The wrapped container is placed in a lower tray and an inverted upper tray is placed over the wrapped container and the parts fastened together to assemble the cover about the wrap container. That assembly can then be sterilised with the plurality of apertures in the main body of the cover allowing the easy passage of steam into and out of the wrapped container. After sterilisation, the assembly can be moved using the handles and the sterilised assembly stored in stacks of similar assemblies in a storage facility. As mentioned above, the outer surfaces of the upper and lower trays present a number of convex ribs, e.g. 382, and a number of concave similarly shaped troughs, e.g. 384. Similarly shaped ribs and troughs are provided between the plurality of apertures either side of the main viewing aperture 380. When stacked, the ribs and troughs of the upper surface of a lower tray can engage with the troughs and ribs respectively of a lower surface of an adjacent upper tray. The ribs and troughs extend generally along the longitudinal axis of the cover and are generally elongate and straight and facilitate the sliding of trays relative to each other in the longitudinal direction. This can facilitate the removal of a tray from within a stack of trays without interrupting the stability of the stack.

Also, the interaction of the ribs and troughs help to prevent sliding of trays within a stack of trays in a direction generally transverse to the longitudinal axis of the cover. For example, if a stack of several assemblies is being carried to or from the store room, then this helps to prevent trays from sliding off the stack in a transverse direction which can otherwise happen.

The interaction between the ribs and troughs, or ridges and grooves, also helps to provide self-alignment of the stacked trays. Further, the grooves provide an air space which improves the effectiveness of the sterilisation. As discussed above, the interaction of the ridges and grooves also provides stability for stacks of trays during transportation.

This cover is quicker and easier to assemble about a wrapped container than the embodiments. Also, it is easily adjustable to accept wrapped containers of different sizes. As would be appreciated, as each tray component is identical, it is only necessary to fabricate a single type of item and then each tray can be used with similarly shaped trays in a modular system in order to provide the cover.

In practice, the tray can be thermoformed from a thermoplastic material, such as polypropylene, and the tray can be thermoformed using a heat based moulding process. A preferred gauge for the polypropylene material is approximately 0.04 to 0.06 inches. Other suitable thermoformable plastics which can be used to fabricate the cover include polyethylene and acrylonitrile butadiene styrene (ABS).

As will be appreciated from the above discussion of the invention, it provides a number of advantages and solves a number of problems associated with wrapped sterilisable medical containers. The cover of the invention helps to retain the wrapping material about the container in a tidy manner which helps to facilitate handling and storage of the wrapped containers. Also, the cover serves to at least partially protect the wrapping material thereby helping to reduce the incidence of damage or tearing of the wrapping material. Further, the covers provide a generally flat surface which further facilitates the storage by stacking of multiple wrapped containers. Further, the covers facilitate access to information about the contents of the wrapped container so that it is easier to ensure that the correct sterilised contents are retrieved from a store when required. Yet further, the integral handle further facilitates the handling and manipulation of the wrap container so there is no need to grasp the wrapping material or try to access any handle on the container as the cover provides an easily accessible handle to a user.

The exact dimensions of the cover, and the position of the various cuts and fold marks in the various embodiments, will depend on the size of container or tray with which it is intended to use the cover. For example, sterilisable containers or trays are generally provided with dimensions of height by width by depth or approximately 5 x 25 x 50, 10 x 25 x 50, 15 x 25 x 50, 5 x 25 x 25 and 10 x 25 x 25 (all measurements in centimetres). It will be appreciated that the cover can be used with other sizes of sterilisation trays or containers as required. Therefore, the dimensions of the cover will generally match the dimensions of the container to be covered, but with some tolerance to accept the sheet or sheets of wrapping material in which the container has been wrapped.

In other embodiments of the invention, the cover can be dimensioned to accept multiple containers therein. For example, a cover can be provided which can accept a stack of individual trays or containers. Additionally or alternatively, covers can be provided with fastening mechanisms which allow the size of the cover to be adjusted depending on the size of the container to be covered. Hence, different height stacks of containers could be received within a single cover.

## Claims

1. An assembly (160) comprising:
a sterilizable medical tray;
a medical device in the medical tray;
wrapping material (150) wrapped around the medical tray; and wherein the assembly (160) further comprises:
a cover (100, 200, 220, 230, 290, 310) around the wrapped medical tray to hold the wrapping material (150) about the medical tray, wherein the cover includes:
a body including an upper part (124) holding the wrapping material against an upper portion of the medical tray, a lower part (120, 260) holding the wrapping material against a lower portion of the medical tray; and
at least one handle (128, 130, 202, 264, 268) extending from the body, and provided as an integral part of the body, and which can be held by a user to move the assembly, and wherein the cover (100) is made from a single sheet (102, 232, 292, 312) of sterilizable material and is assembled about the wrapped sterilizable tray by folding the sheet of material (102, 232, 292, 312) and the medical device is an orthopaedic implant, orthopaedic instrument or orthopaedic tool.

2. The assembly (160) of claim 1, wherein the single sheet of sterilizable material (102) is cardboard.

3. The assembly of claim 1, wherein the single sheet of sterilizable material (232, 292, 312) is a plastic and includes a plurality of apertures (280) to make the body permeable to steam.

4. The assembly (160) of claim 1, wherein the cover (100, 230, 290) includes a fastener (136, 250, 252, 254) for securing the cover (100, 230, 290) about the wrapped sterilizable medical tray.

5. The assembly (160) of claim 1, wherein the single sheet of sterilizable material (102, 232, 292, 312) includes at least one fold formation (104, 234, 236) which defines where the single sheet of sterilizable material can be folded.

6. The assembly (160) of claim 4, wherein the fastener (136) is an adhesive fastener.

7. The assembly of claim 1, wherein the single sheet of sterilizable material (232, 292, 312) includes a plurality of cuts (242, 244) which defines how the single sheet of sterilizable material can be folded to assemble the cover.

8. The assembly of claim 1, wherein the cover (230, 290, 310) includes a first handle (264) at a first end of the body and second handle (268) at a second end of the body.

9. The assembly of claim 4, wherein the fastener (250, 252, 254) is a mechanical fastener.

10. The assembly of claim 1, wherein the cover (290, 310) includes at least one retaining formation (294, 296, 298, 300, 314, 316) to retain the wrapped medical tray within cover.

11. The assembly of claim 10, wherein the or each retaining formation (294, 296, 298, 300, 314, 316) is located toward an open end of the cover (290, 310) to prevent the wrapped medical tray from escaping from the cover via the open end.

12. The assembly of claim 10 or 11, wherein the or each retaining formation (294, 296, 298, 300, 314, 316) is provided as an integral part of the cover (290, 310).

13. The assembly of any of claims 10 to 12, wherein the or each retaining formation (294, 296, 298, 300, 314, 316) is formed by bending or folding a part of the material of the cover (290, 310).

14. A method for covering a sterilizable medical tray wrapped in at least one sheet of wrapping material comprising the steps of:
placing a sterilizable medical tray, having an orthopaedic implant, instrument or tool therein, and wrapped in the wrapping material onto a lower part of a body of the cover to hold the wrapping material against a lower portion of the medical tray; and
folding the body of the cover about the wrapped medical container so that an upper part of the body of the cover holds the wrapping material against an upper portion of the medical tray so as to hold the wrapping material about the medical tray; and
securing the cover about the wrapped medical tray, wherein the cover is made from a single sheet of sterilizable material and includes at least one handle extending from the body, and provided as an integral part of the body, and which can be held by a user to move an assembly of the covered sterilizable medical tray wrapped in at least one sheet of wrapping material.

## Patentansprüche

1. Anordnung (160), umfassend:
ein sterilisierbares medizinisches Tablett;
eine medizinische Vorrichtung auf dem medizinischen Tablett;
Verpackungsmaterial (150), das um das medizinische Tablett geschlagen ist; und
wobei die Anordnung (160) überdies Folgendes umfasst:
eine Abdeckung (100, 200, 220, 230, 290, 310) um das verpackte medizinische Tablett, um das Verpackungsmaterial (150) um das medizinische Tablett herum zu halten, wobei die Abdeckung Folgendes umfasst:
einen Körper, der einen oberen Teil (124) umfasst, der das Verpackungsmaterial gegen einen oberen Abschnitt des medizinischen Tabletts hält, einen unteren Teil (120, 260) umfasst, der das Verpackungsmaterial gegen einen unteren Abschnitt des medizinischen Tabletts hält; und
mindestens einen Griff (128, 130, 202, 264, 268), der sich vom Körper erstreckt und als integraler Bestandteil des Körpers vorgesehen ist und der von einem Benutzer gehalten werden kann, um die Anordnung zu bewegen, und wobei die Abdeckung (100) aus einem einzelnen Bogen (102, 232, 292, 312) sterilisierbaren Materials hergestellt ist und um das verpackte sterilisierbare Tablett herum angeordnet wird, indem der Materialbogen (102, 232, 292, 312) gefaltet wird und die medizinische Vorrichtung ein orthopädisches Implantat, orthopädisches Instrument oder orthopädisches Werkzeug ist.

2. Anordnung (160) nach Anspruch 1, wobei der einzelne Bogen sterilisierbaren Materials (102) Karton ist.

3. Anordnung nach Anspruch 1, wobei der einzelne Bogen sterilisierbaren Materials (232, 292, 312) ein Kunststoff ist und eine Vielzahl von Öffnungen (280) umfasst, um den Körper dampfdurchlässig zu machen.

4. Anordnung (160) nach Anspruch 1, wobei die Abdeckung (100, 230, 290) einen Verschluss (136, 250, 252, 254) umfasst, um die Abdeckung (100, 230, 290) um das verpackte sterilisierbare medizinische Tablett herum zu verschließen.

5. Anordnung (160) nach Anspruch 1, wobei der einzelne Bogen sterilisierbaren Materials (102, 232, 292, 312) mindestens eine Falzformation (104, 234, 236) umfasst, die festlegt, wo der einzelne Bogen sterilisierbaren Materials gefaltet werden kann.

6. Anordnung (160) nach Anspruch 4, wobei der Verschluss (136) ein Klebeverschluss ist.

7. Anordnung nach Anspruch 1, wobei der einzelne Bogen sterilisierbaren Materials (232, 292, 312) eine Vielzahl von Einschnitten (242, 244) umfasst, die festlegen, wie der einzelne Bogen sterilisierbaren Materials gefaltet werden kann, um die Abdeckung anzuordnen.

8. Anordnung nach Anspruch 1, wobei die Abdeckung (230, 290, 310) einen ersten Griff (264) an einem ersten Ende des Körpers und einen zweiten Griff (268) an einem zweiten Ende des Körpers umfasst.

9. Anordnung nach Anspruch 4, wobei der Verschluss (250, 252, 254) ein mechanischer Verschluss ist.

10. Anordnung nach Anspruch 1, wobei die Abdeckung (290, 310) mindestens eine Rückhalteformation (294, 296, 298, 300, 314, 316) umfasst, um das verpackte medizinische Tablett innerhalb der Abdeckung zurückzuhalten.

11. Anordnung nach Anspruch 10, wobei die oder jede Rückhalteformation (294, 296, 298, 300, 314, 316) zu einem offenen Ende der Abdeckung (290, 310) hin angeordnet ist, um zu verhindern, dass das medizinische Tablett über das offene Ende aus der Abdeckung austritt.

12. Anordnung nach Anspruch 10 oder 11, wobei die oder jede Rückhalteformation (294,296,298, 300, 314, 316) als integraler Bestandteil der Abdeckung (290, 310) vorgesehen ist.

13. Anordnung nach Anspruch 10 oder 12, wobei die oder jede Rückhalteformation (294, 296, 298, 300, 314, 316) durch Biegen oder Falten eines Teils des Materials der Abdeckung (290, 310) gebildet wird.

14. Verfahren zum Abdecken eines sterilisierbaren medizinischen Tabletts, das in mindestens einem Bogen Verpackungsmaterial verpackt ist, das folgende Schritte umfasst:
Anordnen eines sterilisierbaren medizinischen Tabletts, das darauf ein orthopädisches Implantat, Instrument oder Werkzeug umfasst und in dem Verpackungsmaterial verpackt ist, auf einem unteren Teil eines Körpers der Abdeckung, um das Verpackungsmaterial gegen einen unteren Abschnitt des medizinischen Tabletts zu halten; und
Falten des Körpers der Abdeckung um den verpackten medizinischen Behälter, sodass ein oberer Teil des Körpers der Abdeckung das Verpackungsmaterial gegen einen oberen Abschnitt des medizinischen Tabletts hält, um das Verpackungsmaterial um das medizinische Tablett herum zu halten; und
Verschließen der Abdeckung um das verpackte medizinische Tablett, wobei die Abdeckung aus einem einzelnen Bogen sterilisierbaren Materials hergestellt ist und mindestens einen Griff umfasst, der sich vom Körper erstreckt und als integraler Bestandteil des Körpers vorgesehen ist und der von einem Benutzer gehalten werden kann, um eine Anordnung des abgedeckten sterilisierbaren medizinischen Tabletts, das in mindestens einem Bogen Verpackungsmaterial verpackt ist, zu bewegen.

## Revendications

1. Ensemble (160) comprenant :
un plateau médical stérilisable ;
un dispositif médical dans le plateau médical ;
du matériau d'enveloppement (150) enveloppé autour du plateau médical ; et dans lequel
l'ensemble (160) comprend en outre :
un couvercle (100, 200, 220, 230, 290, 310) autour du plateau médical enveloppé pour maintenir le matériau d'enveloppement (150) autour du plateau médical, dans lequel le couvercle inclut :
un corps incluant une partie supérieure (124) contenant le matériau d'enveloppement contre une portion supérieure du plateau médical, une partie inférieure (120, 260) contenant le matériau d'enveloppement contre une portion inférieure du plateau médical ; et
au moins une poignée (180, 130, 202, 264, 268) s'étendant depuis le corps, et fournie en tant que partie solidaire du corps, et qui peut être tenue par un utilisateur pour déplacer l'ensemble, et dans lequel le couvercle (100) est composé à partir d'une simple feuille (102, 232, 292, 312) de matériau stérilisable et est assemblé autour du plateau stérilisable enveloppé en pliant la feuille de matériau (102, 232, 292, 312) et le dispositif médical est un implant orthopédique, un instrument orthopédique ou un outil orthopédique.

2. Ensemble (160) selon la revendication 1, dans lequel la simple feuille de matériau stérilisable (102) est du carton.

3. Ensemble selon la revendication 1, dans lequel la simple feuille de matériau stérilisable (232, 292, 312) est un plastique et inclut une pluralité d'ouvertures (280) pour rendre le corps perméable à la vapeur.

4. Ensemble (160) selon la revendication 1, dans lequel le couvercle (100, 230, 290) inclut un élément de fixation (136, 250, 252, 254) pour fixer le couvercle (100, 230, 290) autour du plateau médical stérilisable enveloppé.

5. Ensemble (160) selon la revendication 1, dans lequel la simple feuille de matériau stérilisable (102, 232, 292, 312) inclut au moins une formation de pli (104, 234, 236) qui définit où la simple feuille de matériau stérilisable peut être pliée.

6. Ensemble (160) selon la revendication 4, dans lequel l'élément de fixation (136) est un élément de fixation adhésif.

7. Ensemble selon la revendication 1, dans lequel la simple feuille de matériau stérilisable (232, 292, 312) inclut une pluralité de découpes (242, 244) qui définissent la manière dont la simple feuille de matériau stérilisable peut être pliée pour assembler le couvercle.

8. Ensemble selon la revendication 1, dans lequel le couvercle (230, 290, 310) inclut une première poignée (264) à une première extrémité du corps et une seconde poignée (268) à une seconde extrémité du corps.

9. Ensemble selon la revendication 4, dans lequel l'élément de fixation (250, 252, 254) est un élément de fixation mécanique.

10. Ensemble selon la revendication 1, dans lequel le couvercle (290, 310) inclut au moins une formation de retenue (294, 296, 298, 300, 314, 316) pour retenir le plateau médical enveloppé dans le couvercle.

11. Ensemble selon la revendication 10, dans lequel la ou chaque formation de retenue (294, 296, 298, 300, 314, 316) est située vers une extrémité ouverte du couvercle (290, 310) pour empêcher le plateau médical enveloppé de se déloger du couvercle via l'extrémité ouverte.

12. Ensemble selon la revendication 10 ou 11, dans lequel la ou chaque formation de retenue (294, 296, 298, 300, 314, 316) est fournie en tant que partie solidaire du couvercle (290, 310).

13. Ensemble selon l'une quelconque des revendications 10 à 12, dans lequel la ou chaque formation de retenue (294, 296, 298, 300, 314, 316) est formée en courbant ou pliant une partie du matériau du couvercle (290, 310).

14. Procédé pour recouvrir un plateau médical stérilisable enveloppé dans au moins une feuille de matériau d'enveloppement comprenant les étapes consistant à :
placer un plateau médical stérilisable, ayant un implant, instrument ou outil orthopédique dans celui-ci, et enveloppé dans le matériau d'enveloppement sur une partie inférieure d'un corps du couvercle pour maintenir le matériau d'enveloppement contre une portion inférieure du plateau médical ; et
plier le corps du couvercle autour du récipient médical enveloppé de telle sorte qu'une partie supérieure du corps du couvercle maintient le matériau d'enveloppement contre une portion supérieure du plateau médical de manière à maintenir le matériau d'enveloppement autour du plateau médical ; et
fixer le couvercle autour du plateau médical enveloppé, dans lequel le couvercle est composé à partir d'une simple feuille de matériau stérilisable et inclut au moins une poignée s'étendant depuis le corps, et fournie en tant que partie solidaire du corps, et qui peut être tenue par un utilisateur pour déplacer un ensemble du plateau médical stérilisable recouvert enveloppé dans au moins une feuille de matériau d'enveloppement.
